Europäisches Patentamt

European Patent Office

Office européen des brevets

(19)

(11) Veröffentlichungsnummer: **0 022 540**
**B1**

(12) **EUROPÄISCHE PATENTSCHRIFT**

(45) Veröffentlichungstag der Patentschrift:
**12.10.83**

(51) Int. Cl.³: **A 61 M 31/00,** A 61 D 7/00

(21) Anmeldenummer: **80103879.5**

(22) Anmeldetag: **08.07.80**

(54) Schluckbare Kapsel zur Freisetzung von Substanzen an definierten Orten des Verdauungstraktes.

(30) Priorität: **14.07.79 DE 2928477**

(43) Veröffentlichungstag der Anmeldung:
**21.01.81 Patentblatt 81/3**

(45) Bekanntmachung des Hinweises auf die Patenterteilung:
**12.10.83 Patentblatt 83/41**

(84) Benannte Vertragsstaaten:
**AT BE CH DE FR GB IT LI NL SE**

(56) Entgegenhaltungen:
**EP-A-0 002 776**
**CH-A-337 989**
**US-A-2 130 305**

(73) Patentinhaber: **Battelle-Institut e.V., Am Römerhof 35 Postfach 900 160, D-6000 Frankfurt/Main 90 (DE)**

(72) Erfinder: **Hugemann, Bernhard, Heinrich-Seliger-Strasse 49, D-6000 Frankfurt/Main (DE)**
Erfinder: **Schuster, Otto, Dr., Kelkheimer Strasse 69, D-6232 Bad Soden (DE)**

(74) Vertreter: **Blum, Klaus-Dieter, Dipl.-Ing., Am Römerhof 35, D-6000 Frankfurt/Main 90 (DE)**

Schluckbare Kapsel zur Freisetzung von Substanzen an definierten Orten des Verdauungstraktes

Die Erfindung betrifft eine schluckbare Kapsel zur Freisetzung von Substanzen an definierten Orten des Verdauungstraktes, in der ein heizbares Element mit einer Schmelzsicherung in Verbindung steht, die zur Spannung einer Druckfeder dient, deren Kraft, die nach dem bei Einwirkung eines externen elektromagnetischen Hochfrequenzfeldes erfolgten Erhitzen des Heizelementes und Schmelzen der Schmelzsicherung frei wird, zum Freisetzen der in der Kapsel befindlichen Substanz genutzt wird.

In der Human- und Veterinärmedizin führt die konventionelle orale Madikamenteneinnahme insbesondere dann nicht zu erwünschten Ergebnissen, wenn eine gezielte lokale Behandlung bestimmter Stellen des Magen-Darm-Traktes erforderlich ist. Ein wesentlicher Nachteil besteht unter anderem darin, dass das Medikament durch vorherige Zersetzung bzw. Verdauung an erkrankter Stelle keine oder gar eine schädliche Wirkung entfalten kann. Mit den derzeit verwendeten Methoden können weder Resorptionsbereiche noch Resorptionsintensitäten festgestellt werden. Eine Umhüllung des Präparates, die sich aufgrund ihrer chemischen Zusammensetzung und Schichtdicke nicht sofort auflöst, ist ebenfalls nicht vorteilhaft, da die individuell verschiedenen pH-Werte und Darmaktivitäten eine kontrollierte Verabreichung erschweren. Auch Zuführung von Arzneimitteln durch eine Schlauchsonde stellt eine erhebliche Belastung für den Organismus dar und ist nur in Ausnahmefällen möglich.

In der CH-A-337 989 wird eine schluckbare Kapsel beschrieben, die ein Arzneimittel enthält und dieses bei der Passage des Verdauungstraktes freisetzt. Hierfür wird durch Anwendung eines Hochfrequenzfeldes ein Metallblock aufgeheizt, ein Metallstück geschmolzen und die dabei frei werdende Federkraft zum Öffnen der Kapsel genutzt. Je nach Ankopplung des externen Feldes ist jedoch eine unbestimmte Zeit zur Öffnung der Kapsel erforderlich. Folglich ist es keineswegs sicher, ob die Kapsel sich beim Öffnen noch genau an dem röntgenologisch festgestellten Ort befindet. Ein weiterer wesentlicher Nachteil der bekannten Vorrichtung besteht darin, dass die geschmolzenen Metallstücke das Medikament verschmutzen können.

Der vorliegenden Erfindung liegt daher die Aufgabe zugrunde, eine schluckbare Kapsel zu entwickeln, die eine Substanzfreigabe genau an vorbestimmten Stellen des Verdauungstraktes ermöglicht.

Ferner sollte eine Verschmutzung der Substanz verhindert werden und die Freigabe möglichst ohne Dimensionsvergrösserung der Kapsel erfolgen.

Es hat sich nun gezeigt, dass diese Aufgabe in technisch fortschrittlicher Weise gelöst werden kann, wenn als Heizelement ein Heizdraht verwendet ist, der einen Teil eines aus einer Spule und einem Kondensator gebildeten, aufdie Frequenz des externen Hochfrequenzfeldes abgestimmten Schwingkreises bildet, und wenn in der Kapsel ein abgetrennter Substanzraum vorgesehen ist. Vorteilhafte Ausführungsformen der erfindungsgemässen Kapsel sind in den abhängigen Ansprüchen 2 bis 4 beschrieben.

Die erfindungsgemässe Kapsel setzt auf ein äusseres Signal hin den Wirkstoff frei. In Form und Abmessung entspricht sie einer üblichen Medikamentenkapsel und kann daher geschluckt werden. Bei einem Fassungsvermögen von z.B. 1 ml Wirkstoff beträgt der Aussendurchmesser der vollständigen Kapsel einschliesslich Auslösemechanismus und Wirkstoffraum etwa 12 mm, die Länge ca. 25 mm. Die Kapsel gelangt aus dem Magen in den Darm und wird schliesslich mit dem Stuhl wieder ausgeschieden.

Wird eine solche Kapsel dem Patienten verabreicht, so kann ihre jeweilige Stelle während der Passage des Verdauungstraktes mittels Röntgenstrahlung oder Ultraschall festgestellt werden. Durch Einwirkung eines externen elektromagnetischen oder magnetischen Feldes auf entsprechende Körperpartien des Patienten wird die Kapsel, gewissermassen ferngesteuert, exakt an vorbestimmter Stelle geöffnet und das Medikament freigesetzt. Der Öffnungsmechanismus wird mit einem aus einem Heizdraht, einer Spule und einem Kondensator gebildeten Schwingkreis ausgelöst. Durch richtige Dimensionierung der Spule und des Kondensators wird die Resonanzfrequenz vorbestimmt, so dass bei Einwirkung eines Hochfrequenzfeldes eines auf gleicher Frequenz schwingenden Senders der Heizdraht erwärmt wird. Zur Anbringung des Hochfrequenzfeldes kann ein an sich bekannter Hochfrequenz-Sender verwendet werden, der in der Weise modifiziert ist, dass er eine Einkoppelspule aufweist, die entsprechend dem Körper des Patienten dimensioniert ist. Bei Abstimmung des in der Kapsel befindlichen Schwingungskreises auf z.B. 4 MHz genügt im allgemeinen ein Hochfrequenz-Sender mit ca. 20 Watt HF-Keistung zum Öffnen der Kapsel.

Die erfindungsgemässe Kapsel enthält einen separaten Substanzraum. In einer bevorzugten Ausführungsform wird z.B. die flüssige oder pulver- bzw. kugelförmige Substanz in einen Ballon gefüllt, der in der Kapsel untergebracht ist. Der Öffnungsmechanismus wirkt auf eine Nadel, die den Ballon durchsticht. Nach dem abrupten Zerplatzen des Ballons wird die Substanz durch geeignete Öffnungen, Löcher bzw. Aussparungen in der Kapselhülle freigesetzt.

Im folgenden wird die Erfindung anhand von lediglich einen Ausführungsweg darstellenden Zeichnungen näher erläutert.

Es zeigen in schematischer Vereinfachung

Fig. 1 Anordnung eines Schwingkreises, der innerhalb der Kapsel, angebracht wird;

Fig. 2 eine Ausführungsform, bei der die Substanz sich in einem Ballon befindet und

Fig. 3 die Füllung der in Fig. 2 dargestellten Ausführungsform mit der Substanz.

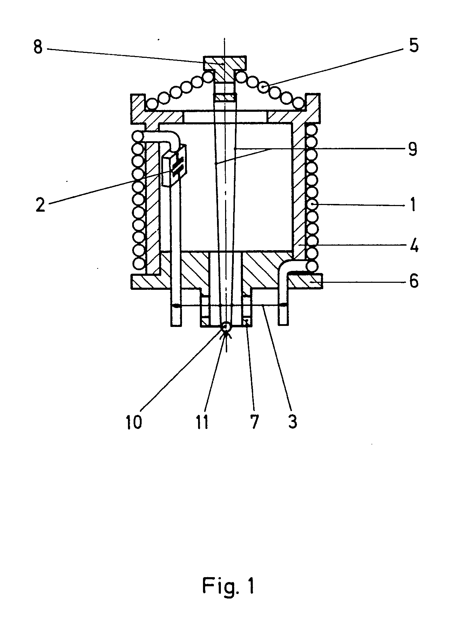

Aus Fig. 1 geht hervor, dass der Schwingkreis aus einer Spule 1, z.B. Kupferdraht, einem Kondensator 2 und einem Heizdraht 3 besteht. Die Schwingkreis-Kupferdrahtspule 1 ist auf dem Spulenkörper 4 angeordnet. Dieser ist so gestaltet, dass er gleichzeitig als Halterung für eine Kelchfeder 5 und den Kondensator 2 dient. Die andere Seite des Spulenkörpers ist mit einem Deckel 6 abgeschlossen, durch den die Anschlussdrähte der Spule 1 und des Kondensators 2 geführt sind. An diesen Drähten ist der Heizdraht 3 angeschlossen und durch eine im Zapfen 7 befindliche Querbohrung geführt. Durch eine weitere Längsbohrung im Zapfen 7 und das Loch im Kunststoffnippel 8 wird ein leicht schmelzender Faden 9, z.B. aus Perlon, hin und hergeführt und über den Knebel 10 durch einen Knoten 11 verbunden. Zuvor wird der Faden 9 jedoch so angezogen, dass die Kelchfeder 5 gespannt ist. Durch Verdrehen des Knebels 10 auf dem Zapfen 7 wird der Faden 9 so justiert, dass beide Fadenschenkel den Heizdraht 3 berühren. Wird nun der Faden 9 durch den Heizdraht 3 durchgeschmolzen, so wird die Kraft der gespannten Kelchfeder 5 wirksam und das Präparat ausgestossen. Alle Kapsel- und Spulenkörperteile werden aus einem Material gefertigt, das den medizinischen Forderungen entspricht.

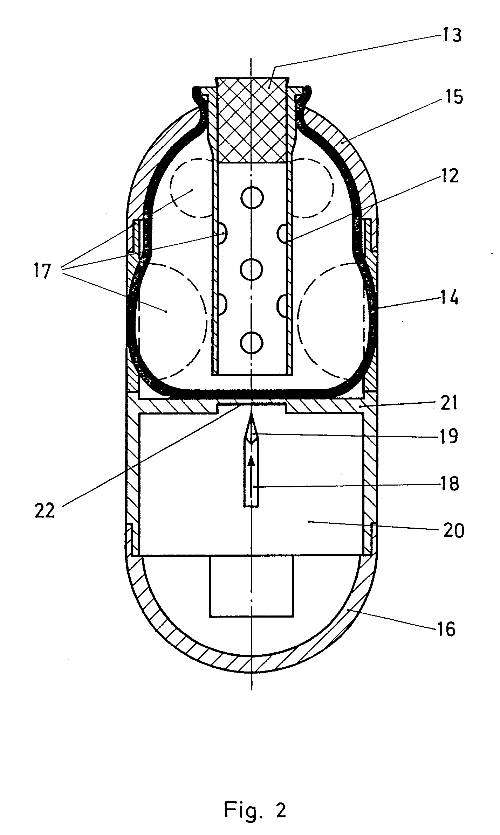

Aus Fig. 2 geht hervor, dass die Kapsel eine rohrförmige Halterung 12 aufweist, die mit einem Gummistopfen 13 verschlossen ist. Ein Latex-Formteil 14, das den Ballon bildet, ist an der Halterung 12 montiert und innerhalb der Verschlussklappe 15 der Kapsel untergebracht. Die Halterung 12, Verschlusskappe 15 bzw. Kapselgehäuse 16 sind an geeigneten Stellen mit Löchern bzw. Aussparungen 17 versehen, die ein schnelles Auslaufen bzw. Entleeren der Substanz ermöglichen. Eine Nadel 18, die vorzugsweise mit zusätzlichen Schneidkanten 19 versehen ist, ist zentrisch in dem Antriebsmechanismus 20 angeordnet. Die zusätzlichen Schneidkanten 19 ermöglichen einen nicht nur stechenden, sondern auch schneidenden Effekt, wodurch ein Zerplatzen des Ballons 14 und eine abrupte Freigabe der Substanz gesichert ist. Die Nadelspitze befindet sich unter der Trennwand 21 der Kapsel. Die Trennwand 21 enthält in der Mitte bzw. im Durchstichbereich der Nadel eine Öffnung 22, die mit einer Folie abgeschlossen sein kann. Nach Auslösung der Nadel 18 durchsticht diese zunächst die Folie im Bereich der Öffnung 22 und dann die Latexhülle 14, wodurch diese abrupt zerplatzt und die eingefüllte Substanz durch die Aussparungen bzw. Löcher in der Hülle 15 freigibt.

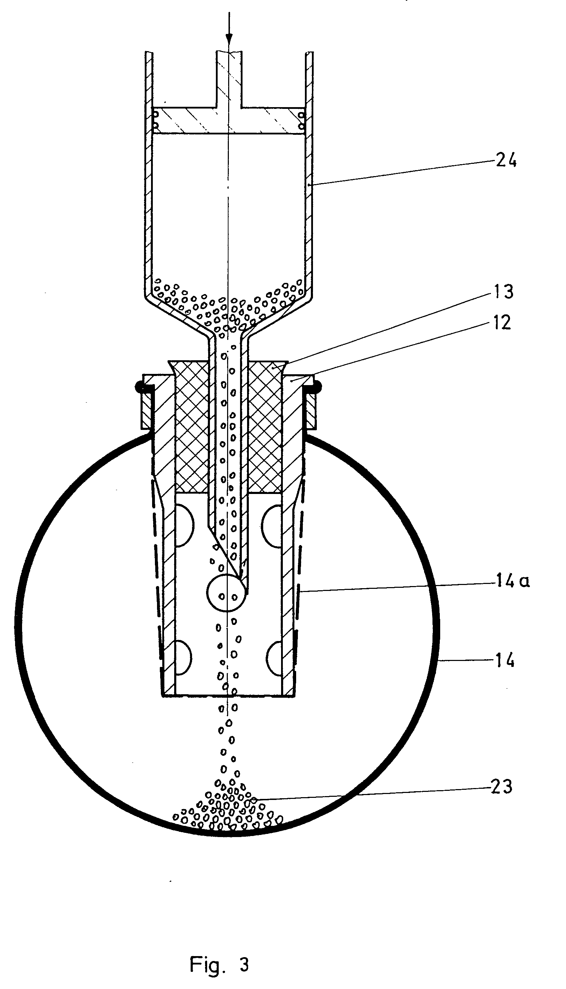

In Fig. 3 wird schematisch die Einfüllung der Substanz in eine in Fig. 2 dargestellte Vorrichtung gezeigt. Sowohl flüssige als auch pulverförmige Substanzen können in den Ballon 14 eingebracht werden. Bei Füllung des Ballons mit pulverförmigem Wirkstoff 23 muss in den Ballon 14 zuerst Luft eingeblasen werden, so dass die Substanz nach dem Prinzip der Sanduhr aus der Injektionsspritze 24 in den Ballon 14 rieseln kann. Damit der Ballon im Wirkungsbereich der Nadel 18 bleibt, falls geringe Substanzmengen verwendet werden und der Ballon nicht optimal ausgedehnt ist, ist die Halterung 12 vorzugsweise 13 mm bis 14 mm lang. Der Stopfen 13 der rohrförmigen Halterung 12 ist vorzugsweise aus einem solchen Material angefertigt, dass die Einstichöffnung der Kanüle 24 sich nach dem Entfernen der Kanüle selbsttätig wieder schliesst.

## Patentansprüche

1. Schluckbare Kapsel zur Freisetzung von Substanzen an definierten Orten des Verdauungstraktes, in der ein heizbares Element (3) mit einer Schmelzsicherung (9) in Verbindung steht, die zur Spannung einer Druckfeder (5) dient, deren Kraft, die nach dem bei Einwirkung eines externen elektromagnetischen Hochfrequenzfeldes erfolgten Erhitzen des Heizelementes (3) und Schmelzen der Schmelzsicherung (9) frei wird, zum Freisetzen der in der Kapsel (15, 16) befindlichen Substanz genutzt wird, dadurch gekennzeichnet, dass als Heizelement ein Heizdraht (3) verwendet ist, der einen Teil eines aus einer Spule (1) und einem Kondensator (2) gebildeten, auf die Frequenz des externen Hochfrequenzfeldes abgestimmten Schwingkreises (1, 2, 3) bildet, und dass in der Kapsel ein abgetrennter Substanzraum (14) vorgesehen ist.

2. Vorrichtung nach Anspruch 1, dadurch gekennzeichnet, dass die Substanz in einem Ballon (14) untergebracht ist, der sich innerhalb der mit Löchern und Aussparungen (17) versehenen Kapselhülle (15) befindet, und dass durch die Kraft der Druckfeder (5) eine Nadel (18) so bewegt wird, dass sie den Ballon (14) durchsticht und zum Zerplatzen bringt.

3. Vorrichtung nach Anspruch 2, dadurch gekennzeichnet, dass die Nadelspitze mit zusätzlichen Schneidkanten (19) versehen ist.

4. Vorrichtung nach Anspruch 2 oder 3, dadurch gekennzeichnet, dass eine rohrförmige und mit Löchern (17) versehene Halterung (12) mit dem oberen Teil des Ballons (14) und der Kapselhülle (15) verbunden und von oben luftdicht verschlossen ist, und die Halterung aufgrund geeigneter Dimensionierung den Ballon (14) unabhängig von der eingefüllten Substanzmenge im Stechbereich der Nadel (18) hält.

## Claims

1. Swallowable capsule for the release of substances at defined locations in the alimentary tract in which a heating element (3) is combined with a fuse (9) which serves to bend a compression spring (5), the force of the compression spring which is released after the heating of the heating element (3) and blowing out of the fuse (9) by means of an external electromagnetic field is used to discharge the substance contained in the capsule (15, 16) characterized in that a resistance wire (3) is used as heating element, the resistance wire being a part of a resonant circuit (1, 2, 3) which is tuned to the frequency of the electromagnetic field and that a separate chamber in the capsule is provided for the substance.

2. Device according to claim 1 wherein the substance is contained in a balloon (14) within the capsule enclosure (15) which is provided with holes and slots (17) and wherein a needle (18) is moved by the force of the compression spring (5) in such a way as to pierce and burst the balloon (14).

3. Device according to claim 2 wherein the tip of the needle is provided with additional cutting edges (19).

4. Device according to claim 2 to 3 wherein a tubular mounting (12) which is provided with holes (17) is connected with the upper part of the balloon (14) and the capsule enclosure (15) and sealed airtight from above and wherein the mounting holds the balloon (14) because of its appropriate dimensioning in the piercing area of the needle (18) irrespective of the quantity of substance introduced.

## Revendications

1. Capsule avalable permettant de libérer des substances à des endroits définis du transit digestif, capusle dans laquelle un élément susceptible d'être chauffé (3) est en liaison avec une sécurité fusible (9) servant à contraindre un ressort de compression (5), dont l'action, libérée après l'échauffement de l'élément susceptible d'être chauffé (3), survenu sous l'action d'un champ externe électromagnétique de haute fréquence, et la fusion de la sécurité fusible (9), est utilisée pour la libération de la substance se trouvant dans la capsule (15, 16), capsule caractérisée en ce qu'un fil chauffant (3) est utilisé comme élément susceptible d'être chauffé, ce fil chauffant constituant une partie d'un circuit oscillant (1, 2, 3) constitué d'une bobine (1) et d'un condensateur (2) et accordé sur la fréquence du champ externe haute fréquence, tandis qu'il est prévu dans la capsule un espace séparé (14) pour la substance.

2. Capsule selon la revendication 1, caractérisée en ce que la substance est logée dans un ballon (14) se trouvant à l'intérieur de l'enveloppe (15) de la capsule, muni de trous et d'évidements (17), tandis que sous l'action du ressort de compression (5), une aiguille (18) est mûe de façon qu'elle perce le ballon (14) et le fait éclater.

3. Capsule selon la revendication 2, caractérisée en ce que la pointe de l'aiguille est munie d'arêtes de coupe (19) supplémentaires.

4. Capsule selon l'une quelconque des revendications 2 ou 3, caractérisée en ce qu'un support tubulaire (12) et muni de trous (17) est relié à la partie supérieure du ballon (14) et de l'enveloppe (15) de la capsule en étant fermé par le haut de façon étanche à l'air, tandis que ce support grâce à son dimensionnement approprié, maintient le ballon (14), indépendamment de la quantité de substance introduite dans celui-ci, dans la zone de percement de l'aiguille (18).

Fig. 1

Fig. 2

Fig. 3